(19) **Europäisches Patentamt**

**European Patent Office**

**Office européen des brevets**

(11) Veröffentlichungsnummer: **0 215 376**
**B1**

(12) # EUROPÄISCHE PATENTSCHRIFT

(45) Veröffentlichungstag der Patentschrift:
**24.05.89**

(21) Anmeldenummer: **86112186.1**

(22) Anmeldetag: **03.09.86**

(51) Int. Cl.⁴: **C07H 5/02**, C07H 13/04,
C07H 15/18

(54) **Verfahren zur Herstellung von am Sauerstoff geschützten Glycosylfluoriden.**

(30) Priorität: **14.09.85 DE 3532883**
**01.08.86 DE 3626028**

(43) Veröffentlichungstag der Anmeldung:
**25.03.87 Patentblatt 87/13**

(45) Bekanntmachung des Hinweises auf die Patenterteilung:
**24.05.89 Patentblatt 89/21**

(84) Benannte Vertragsstaaten:
**AT BE CH DE FR GB IT LI NL SE**

(56) Entgegenhaltungen:
**CHEMICAL ABSTRACTS, Band 73, Nr. 7, 17.
August 1970, Seite 356, Zusammenfassung Nr. 35645y,
Columbus, Ohio, US; K. IGARASHI et al.: "Reaction of
glycosyl chlorides with silver tetrafluoroborate"
CARBOHYDRATE RESEARCH, Band 132, Nr. 2,
September 1984, Seiten 339-341, Elsevier Science
Publishers B.V., Amsterdam, NL; Y.V. VOZNIJ et al.:
"Silver tetrafluoroborate as an effective catalyst for the
anomerisation of glycosyl fluorides"
CHEMICAL ABSTRACTS, Band 80, Nr. 17, 29. April 1974,
Seite 442, Zusammenfassung Nr. 96243g, Columbus,
Ohio, US; K. BOCK et al.: "Reaction of sugar esters
with hydrogen fluoride. XII. Derivatives of
D-galactofuranose and D-talofuranose"
CHEMICAL ABSTRACTS, Band 95, Nr. 5, 3. August 1981,
Seite 799, Zusammenfassung Nr. 43491q, Columbus,
Ohio, US; Ya.V. VOZNYI et al.: "New reagent for the
synthesis of glycosyl fluorides"**

(73) Patentinhaber: **HOECHST AKTIENGESELLSCHAFT,
Postfach 80 03 20, D-6230 Frankfurt am Main 80(DE)**

(72) Erfinder: **Thiem, Joachim, Prof.-Dr., Asbeckweg 31,
D-4400 Münster(DE)**
Erfinder: **Deger, Hans-Matthias, Dr., Am Rheingauer
Weg 8, D-6238 Hofheim am Taunus(DE)**
Erfinder: **Fritsche-Lang, Wolfram, Dr., Rhönstrasse 7,
D-6140 Bensheim(DE)**
Erfinder: **Kreuzer, Matthias, Dr., Im Münchfeld 8,
D-6500 Mainz(DE)**

**Beschreibung**

Am Sauerstoff geschützte, d.h. acylierte oder alkylierte Glycosylfluoride sind außerordentlich nützliche Glycosylierungsreagentien in der Kohlenhydrat-Chemie. Es ist bekannt, daß man diese Verbindungen herstellen kann, in dem man z.B.O-perracylierte Monosaccharide mit Fluorwasserstoff umsetzt oder geschützte Saccharide mit freier anomerer Hydroxylgruppe mit Fluorierungsmitteln wie Pyridin-Polyhydrofluorid oder Diethylaminoschwefeltrifluorid (DAST) reagieren läßt. Nach dem Stand der Technik, der zusammenfassend z.B. aus Arbeiten von A. A. E. Penglis (Adv. Carbohydr. Chem. Biochem. 38, 195-285 (1981)) oder A. B. Foster und J. H. Westwood (Pure Appl. Chem. 35, 147-168 (1973)) hervorgeht, entstehen hierbei jedoch fast stets die α-Anomeren. Die präparativ mindestens ebenso interessanten β-Anomeren lassen sich auf diese Weise nicht herstellen, sondern müssen im allgemeinen durch eine Umhalogenierung aus den entsprechenden Chlor- oder Bromverbindungen gewonnen werden. Als Reagens ist hierfür das kostspielige und zudem hydrolyseempfindliche Silberfluorid bekannt. So wird z. B. 2,3,4,6-Tetra-O-acetyl-β-D-glucopyranosylfluorid durch Umsetzung von Acetobromglucose mit AgF nach A. Bertho (Ber. Dtsch. Chem. Ges. 63, 836 (1930)) hergestellt. Auch als Reagens ist 2.4.6-Trimethylpyridin Hydrofluorid bekannt (Chem. Abs. 95:43491q (1989)).

Es zeigt sich also, daß der Stand der Technik zur Herstellung von am Sauerstoff geschützten Glycosylfluoriden entweder vom Einsatz von Fluorwasserstoff, einem schwierig zu handhabenden Reagens, gekennzeichnet ist oder von präparativem Aufwand, der zur Herstellung geeigneter Ausgangsstoffe, nämlich von selektiv in 1-Stellung ungeschützten Sacchariden getrieben werden muß, um sie anschließend mit ebenfalls nicht unproblematischen Fluorierungsmitteln wie Pyridin-Polyhydrofluorid oder DAST umsetzen zu können, oder von hohen Kosten, die mit dem Einsatz von Silberfluorid verbunden sind.

Es wurde nun überraschend gefunden, daß eine Umhalogenierung eines mindestens in 2-Stellung an Sauerstoff geschützten, d. h. acylierten oder alkylierten Glycosylhalogenids auch mit Hilfe von Alkalihydrogendifluoriden gelingt, wenn in Gegenwart eines polar-aprotischen Lösungsmittels gearbeitet wird. Wie es von den Umhalogenierungen mittels Silberfluorid bereits bekannt ist (vgl. A. A. E. Penglis, loc. cit.), entsteht auch hier aus einem mindestens in 2-Stellung O-acylierten Substrat das 1,2-trans-Fluorid. Bei O-alkylierten Substraten erfolgt hingegen bereits in situ in der Regel eine Anomerisierung in die cis-Form. Aber auch bei der Umhalogenierung von O-acylierten Substraten kann man häufig die cis-Form erzeugen, wenn man zusätzlich zum Alkalihydrogendifluorid unterstöchiometrische Mengen eines anorganischen Fluorids mit Lewissäure-Charakter einsetzt.

Gegenstand der Erfindung ist folglich ein Verfahren zur Herstellung von 1,2-trans- bzw. 1,2-ciskonfigurierten Glycosylfluoriden, die mindestens in 2-Stellung O-acyliert oder O-alkyliert sind, durch Umhalogenierung, das dadurch gekennzeichnet ist, daß man die entsprechend O-acylierten oder O-alkylierten Glycosylhalogenide in einem polar - aprotischen Lösungsmittel mit Alkalihydrogendifluoriden umsetzt und gegebenenfalls unter Zusatz unterstöchiometrischer Mengen eines anorganischen Fluorids mit Lewissäure-Charakter anomerisiert. Das Halogenatom in den beim erfindungsgemäßen Verfahren einzusetzenden, am Sauerstoff geschützten Glycosylhalogeniden besitzt ein Atomgewicht von mindestens 35 und ist bevorzugt Chlor oder Brom.

Die beim erfindungsgemäßen Verfahren einzusetzenden Glycosylhalogenide sind mindestens in 2-Stellung O-acyliert oder O-alkyliert. Es ist jedoch bevorzugt, solche Glycosylhalogenide zu verwenden, deren sämtliche Hydroxylgruppen geschützt, die also peracyliert oder peralkyliert sind. Als Acylreste seien z.B. Acetyl, Chloracetyl, Bromacetyl, Propionyl, Butyryl, Pivaloyl sowie Benzoyl, p-Methoxybenzoyl und o- und p-Nitrobenzoyl genannt. Als Alkylreste sind Gruppen der allgemeinen Formel $-CR^1R^2R^3$ zu betrachten, in denen die Substituenten $R^1$, $R^2$ und $R^3$ gleich oder verschieden sind und Wasserstoff, Alkyl, Alkenyl mit jeweils bis zu 20 C-Atomen bedeuten können sowie Aryl oder ein mehrkerniges aromatisches System mit bis zu 25 C-Atomen, die jeweils am Kern noch Reste mit ein oder mehreren elektronenziehenden oder -schiebenden Substituenten wie Methoxy-, Methyl-, Halogen-, Nitro- oder Nitril-Gruppen enthalten können.

Die genannten, als Ausgangsstoffe verwendbaren, am Sauerstoff geschützten Glycosylhalogenide können sich von Monosacchariden wie Glucose, Galactose, Mannose, Gulose, Talose, Allose, Altrose usw., einschließlich der $C_5$-Aldosen wie Xylose, Ribose usw. ableiten sowie von Oligosacchariden, insbesondere Disacchariden wie Lactose, Maltose, Cellobiose, Gentiobiose, oder auch von höheren Oligosacchariden wie Maltotriose oder Maltotetraose.

Unter den erfindungsgemäß zum Zweck der Umhalogenierung einsetzbaren Alkalihydrogendifluoriden wird das Kaliumhydrogendifluorid bevorzugt.

Als polar - aprotische Lösungsmittel für das erfindungsgemäße Verfahren seien insbesondere die Nitrile genannt, sofern diese als Solventien Verwendung finden, wie z. B. Aceto-, Propio- oder Benzonitril, ferner Nitrokohlenwasserstoffe wie Nitromethan, Nitrobenzol und Nitrotoluol sowie deren Mischungen mit unpolaren aprotischen Lösungsmitteln, also z.B. Gemische von Acetonitril mit Dichlormethan oder Chloroform.

Als anorganische Fluoride mit Lewissäure-Charakter kann z.B. das $BF_3$ oder das $SnF_4$, $ZrF_4$, oder aber, bevorzugt, das $TiF_4$ beim erfindungsgemäßen Verfahren zugesetzt werden, wobei diese Lewissäuren insbesondere in weniger als 25 % der stöchiometrischen Menge verwendet werden.

Die erfindungsgemäße Umhalogenierung wird im allgemeinen bei Atmosphärendruck, vorteilhaft zwischen 10°C und 100°C, bevorzugt zwischen 40°C und 80°C, durchgeführt. Die Anwendung von Über- oder Unterdruck ist möglich, aber nicht bevorzugt.

Das erfindungsgemäße Verfahren wird durch die im folgenden angeführten Ausführungsbeispiele illustriert, aber nicht eingeschränkt.

<u>Beispiele</u>

1) Herstellung von 2,3,4,6-Tetra-O-acetyl-β-D-glucopyranosylfluorid

10,0 g (24,8 mmol) 2,3,4,6-Tetra-O-acetyl α-D-glucopyranosylbromid wurden mit 10,0 g (128 mmol) Kaliumhydrogendifluorid in 50 ml absol. Acetonitril unter Rückfluß erhitzt. Nach 24 h wurde filtriert, das Lösungsmittel im Vakuum abgezogen und der Rückstand in Chloroform aufgenommen. Nach dreimaligem Waschen mit Wasser und Trocknen über Magnesiumsulfat erhielt man 7,4 g Rohprodukt, in dem nach Dünnschichtchromatographie (DC) (Toluol/Essigsäureethylester, 1:1) neben 2,3,4,6-Tetra-O-acetyl-β-D-glucopyranosylfluorid noch Spuren von Hydrolyseprodukten enthalten waren. Die Reinigung· erfolgte durch Umkristallisation aus Diethylether.
Ausbeute 5,9 g (70 %), Schmp. 88-89°C, $[\alpha]_D = +20$ (c= 1,0 in Chloroform) [A. Bertho, l.c.; Schmp 89°C, $[\alpha]_D = +21,9$ (Chloroform)].

2) Herstellung von 2,3,4,6-Tetra-O-acetyl-β-D-galactopyranosylfluorid

10,0 g (24,3 mmol) 2,3,4,6-Tetra-O-acetyl-α-D-galactopyranosylbromid wurden in 100 ml wasserfreiem Acetonitril mit 10,0 g (128 mmol) Kaliumhydrogendifluorid 6 h unter Rückfluß erhitzt. Anschließend wurde filtriert, das Lösungsmittel im Vakuum abgezogen und der Rückstand in Methylenchlorid aufgenommen. Nach Filtration über eine kurze Kieselgelsäule und Einengen der Lösung konnte das Produkt aus Ether kristallisiert werden.
Ausbeute 6,1 g (72 %), Schmp. 100°c, $[\alpha]_D^{20} =$ =+17,5 (c= 1,03 in CHCl$_3$) [F. Micheel et al., Chem. Ber.<u>88</u>,475 (1955)] Schmp. 98-99°C, $[\alpha]_D^{18} = +22$ (CH$_3$OH)]. ¹H-NMR (CDCl$_3$): δ = 5,26 (dd, 1-H), $J_{1,2} = 7,0$, $J_{1,F} = 50,0$ Hz.

3) Herstellung von 2,3,4,6-Tetra-O-acetyl-α-D-mannopyranosylfluorid

8,7 g (21,2 mmol) 2,3,4,6-Tetra-O-acetyl-α-D-mannopyranosylbromid wurden in 30 ml wasserfreiem Acetonitril gelöst und mit 5,0 g (64 mmol) Kaliumhydrogendifluorid unter Rückfluß erhitzt. Nach 3 Stunden war kein Ausgangsmaterial mehr nachweisbar (DC: Dichlormethan/Ether, 9:1). Die Lösung wurde filtriert, eingeengt und das Rohprodukt aus Ether umkristallisiert. Ausbeute 5,74 g (77 %) Schmp. 65-67°C, $[\alpha]_D^{20} = +21,2$ (c= 1,07 in Chloroform) [A. Bertho, l.c.: Schmp. 68-69°C, $[\alpha]_D^{20} = +21,5$ (Chloroform)]. ¹H-NMR (CDCl$_3$): δ = 5,58 (dd, 1-H), $J_{1,2} = 1,9$, $J_{1,F} = 48,4$ Hz; [L.D. Hall et al.,

Can. J. Chem. <u>47</u>,1 (1969): δ= 5,57 (dd, 1-H), $J_{1,2} = 1,7$, $J_{1,F} = 48,6$ Hz].

4) In situ Anomerisierung von 2,3,4,6-Tetra-O-acetyl-β-D-galactopyranosylfluorid:

10,0 g (24,3 mmol) 2,3,4,6-Tetra-O-acetyl-α-D-galactopyranosylbromid wurden mit 10,0 g (128 mmol) Kaliumhydrogendifluorid und 0,5 g (4 mmol) Titantetrafluorid in 50 ml sorgfältig getrocknetem Acetonitril 10 Stunden unter Rückfluß erhitzt. Die im Laufe der Reaktion dunkel gefärbte Lösung wurde filtriert, eingeengt, der Rückstand in Methylenchlorid aufgenommen und wiederum über 20 g Kieselgel filtriert. Das Rohprodukt wurde chromatographisch (Kieselgel, n-Hexan/Essigester, 2:1) gereinigt.
Ausbeute 2,7 g (32%) an 2,3,4,6-Tetra-O-acetyl-α-D-galactopyranosylfluorid. ¹H-NMR (CDCl$_3$): δ = 5,81 (dd, 1-H), $J_{1,2} = 2,7$, $J_{1,F} = 52,8$ Hz.

5) Herstellung von 2,3,4,6-Tetra-O-benzyl-α-D-glucopyranosylfluorid:

3,0 g (5,5 mmol) 2,3,4,6-Tetra-O-benzyl-D-glucopyranose wurden in 30 ml wasserfreiem Methylenchlorid gelöst und mit 1,45 ml (2,11 g; 16,5 mmol) Oxalsäuredichlorid sowie 0,3 ml Dimethylformamid bei Raumtemperatur gerührt. Nach einer Stunde wurde die Lösung im Hochvakuum eingeengt, der Rückstand in 10 ml Methylenchlorid aufgenommen und über ca. 10 g Kieselgel filtriert. Erneutes Einengen ergab 2,48 g (80%) 2,3,4,6-Tetra-O-benzyl-α-D-glucopyranosylchlorid als farblosen Sirup. Man nahm das Rohprodukt in 20 ml wasserfreiem Acetonitril auf und erhitzte mit 3,0 g (38 mmol) Kaliumhydrogenfluorid (24 h bei 100°C über P$_2$O$_5$ getrocknet) 8 Stunden unter Rückfluß. Das Rohprodukt konnte säulenchromatographisch an Kieselgel (Methylenchlorid/Ether, 20 : 1) gereinigt werden.
Ausbeute: 1,1 g (47%) 2,3,4,6-Tetra-O-benzyl-α-D-glucopyranosylfluorid.

**Patentansprüche**

1. Verfahren zur Herstellung von 1,2-trans- bzw. -cis-Glycosylfluoriden, die mindestens in 2-Stellung O-acyliert oder O-alkyliert sind, durch Umhalogenierung, dadurch gekennzeichnet, daß man die entsprechend O-acylierten oder O-alkylierten Glycosylhalogenide in einem polar-aprotischen Lösungsmittel mit Alkalihydrogendifluorid umsetzt und gegebenenfalls durch Zusatz unterstöchiometrischer Mengen eines anorganischen Fluorids mit Lewissäure-Charakter anomerisiert.

2. Verfahren nach Anspruch 1, dadurch gekennzeichnet, daß als Glycosylhalogenid ein Chlorid oder Bromid eingesetzt wird.

3. Verfahren nach Anspruch 1 oder 2, dadurch gekennzeichnet, daß Glycosylhalogenide eingesetzt werden, deren sämtliche Hydroxylgruppen geschützt sind.

4. Verfahren nach einem oder mehreren der Ansprüche 1 bis 3, dadurch gekennzeichnet, daß Kaliumhydrogendifluorid als Alkalihydrogendifluorid eingesetzt wird.

5. Verfahren nach einem oder mehreren der Ansprüche 1 bis 4, dadurch gekennzeichnet, daß die anorganischen Fluoride mit Lewissäure-Charakter in weniger als 25% der stöchiometrischen Menge verwendet werden.

6. Verfahren nach einem oder mehreren der Ansprüche 1 bis 5, dadurch gekennzeichnet, daß TiF$_4$ als anorganisches Fluorid mit Lewissäure-Charakter eingesetzt wird.

## Claims

1. A process for the preparation of a 1,2-trans- or -cisglycosyl fluoride which is 0-acylated or 0-alkylated at least in the 2-position, by transhalogenation, which comprises reacting the corresponding 0-acylated or 0-alkylated glycosyl halide with an alkali metal hydrogen difluoride in a polar-aprotic solvent and, if desired, anomerizing the product by addition of less than the stoichiometric amount of an inorganic fluoride with a Lewis acid character.

2. The process as claimed in claim 1, wherein a chloride or bromide is employed as the glycosyl halide.

3. The process as claimed in claim 1 or 2, wherein a glycosyl halide in which all the hydroxyl groups are protected is employed.

4. The process as claimed in any one of claims 1 to 3, wherein potassium hydrogen difluoride is employed as the alkali metal hydrogen difluoride.

5. The process as claimed in any one of claims 1 to 4, wherein the inorganic fluoride with a Lewis acid character is used in less than 25% of the stoichiometric amount.

6. The process as claimed in any one of claims 1 to 5, wherein TiF$_4$ is employed as the inorganic fluoride with a Lewis acid character.

## Revendications

1. Procédé pour préparer des fluorures de glycosyles trans-1,2 ou cis-1,2 qui sont 0-acylés ou 0-alkylés au moins à la position 2, par transhalogénation, procédé caractérisé en ce qu'on fait réagir les halogénures de glycosyles 0-acylés ou 0-alkylés correspondants, dans un solvant aprotique polaire, avec un hydrogénodifluorure de métal alcalin, et éventuellement on anomérise par addition de quantités substoechiométriques d'un fluorure inorganique à caractère d'acide de Lewis.

2. Procédé selon la revendication 1, caractérisé en ce qu'on utilise, comme halogénure de glycosyle, un chlorure ou un bromure.

3. Procédé selon l'une des revendications 1 et 2, caractérisé en ce qu'on utilise des halogénures de glycosyles dont tous les radicaux hydroxy sont protégés.

4. Procédé selon l'une quelconque des revendications 1 à 3, caractérisé en ce qu'on utilise l'hydrogénodifluorure de potassium comme hydrogénodifluorure de métal alcalin.

5. Procédé selon l'une quelconque des revendications 1 à 4, caractérisé en ce que les fluorures inorganiques à caractère d'acide de Lewis sont utilisés en une quantité inférieure à 25% de la quantité stoechiométrique.

6. Procédé selon l'une quelconque des revendications 1 à 5, caractérisé en ce qu'on utilise TiF$_4$ comme fluorure inorganique à caractère d'acide de Lewis.